Europäisches Patentamt

European Patent Office

Office européen des brevets

(19)

(11) **EP 1 413 675 A1**

(12) **EUROPEAN PATENT APPLICATION**

(43) Date of publication:
**28.04.2004 Bulletin 2004/18**

(51) Int Cl.⁷: **D21H 21/04**

(21) Application number: **03005362.3**

(22) Date of filing: **12.03.2003**

| | |
|---|---|
| (84) Designated Contracting States:<br>**AT BE BG CH CY CZ DE DK EE ES FI FR GB GR**<br>**HU IE IT LI LU MC NL PT RO SE SI SK TR**<br>Designated Extension States:<br>**AL LT LV MK RO**<br><br>(30) Priority: **21.10.2002 CN 02146912**<br><br>(71) Applicant: **Yuen Foong Yu Paper MFG Company,**<br>**Limited**<br>**Taipei, Taiwan (TW)** | (72) Inventor: **Wang, Jihn-Yuh**<br>**Taipei 104 (TW)**<br><br>(74) Representative: **Behnisch, Werner, Dr.**<br>**Reinhard-Skuhra-Weise & Partner,**<br>**Patentanwälte,**<br>**Postfach 44 01 51**<br>**80750 München (DE)** |

(54) **Method for reducing slime production and its composition**

(57) A method for reducing a production of a slime in a papermaking process is provided. The method includes steps of adding a dispersing agent into the slime to obtain a first mixture, mixing and culturing the mixture, adding an antagonistic into the first mixture to obtain a second mixture, mixing and culturing the second mixture for reducing the production of the slime, and re-adding the antagonistic into the mixed and cultured second mixture after a specific time period for further reducing the production of the slime.

EP 1 413 675 A1

**Description**

The present invention relates to a method for reducing a production of a slime and a mixture making the same, and more particularly to a method and mixture for reducing a production of a slime by utilizing a dispersing agent and a benefit microorganism having an inhibition/killing ability.

During a papermaking process, the waste pulp is always recycled. However, because a starch and a coating in the waste paper provide an excellent nutrition source, it always believes that they are the main contaminating source for the microorganisms including bacteria and fungus. Otherwise, for reducing a consumption of the water, the papermaking factory usually adopts an airtight water cycle system. However, this condition also provides a favorable growth conditions for every kind of microorganism, e.g., temperature, pH, and nutrition. Consequently, a multiplicity and growth of the microorganism will be more intensified so as to cause a problem of an excess of microorganism. And a formation of the slime at the wet end is a very serious problem in the papermaking factory.

A slime produced at the wet end during the papermaking process will cause the problems of foul smell, paper break and holey, and foxing. All these problems will seriously influence the paper quality and then cause a business trouble which will not only increase the cost owing to the indemnity, but also destroy the reputation.

For preventing the production of the slime, most papermaking factories mainly utilize an organic biocide to reduce the amount of the slime producing microorganisms in the papermaking process and further reduce the slime production so as to solve the problems caused by the slime, e.g., paper holey, foxing, and paper break. However, because the chemical synthesized biocides all are potentially or immediately poisoned to the environment, human beings, and animals, and also because the environmental consciousness is promoted, the standards for how to use and dispose the harmful chemicals are stricter and stricter. Consequently, looking for a non-poisonous and unharmful natural preventing method is the most important challenge so far. And, a research of a mixture which utilizes a local screened antagonistic is one topic thereof.

Because of the technical defects described above, the applicant keeps on carving unflaggingly to develop a "method for reducing slime production and mixture making the same" through wholehearted experience and research.

It is therefore an object of the present invention to reduce the slime production during the papermaking process so as to solve the problems caused by slime.

It is a further object of the present invention to add a benefit microorganism, which dose not cause the slime, into a system consisting white water and substances for preventing or reducing the production of slime.

It is an additional object of the present invention to add a dispersing agent which has an ability of inhibiting the adhesion between the microorganism and the additives during the papermaking process so as to achieving the purpose of inhibiting or reducing a production of a slime.

In accordance with one aspect of the present invention, a method for reducing a production of a slime in a papermaking process includes steps of adding a dispersing agent into the slime to obtain a first mixture, mixing and culturing the mixture, adding an antagonistic into the first mixture to obtain a second mixture, mixing and culturing the second mixture for reducing the production of the slime, and re-adding the antagonistic into the mixed and cultured second mixture after a specific time period for further reducing the production of the slime.

Preferably, the slime is produced during the papermaking process.

Preferably, the antagonistic is *Streptomyces bikiniensis.*

Preferably, the antagonistic is amounted to $10^7$/mL after being cultivated for 24 hrs.

Preferably, the dispersing agent is selected from a group consisting of a Lignosulfonate (B100), a Di-alkyl sulfosuccinate (S100), a Nonionic surfactants (H40), a Polyethylene glycol (P100), a Nonionic surfactants (Bu200).

Preferably, the specific time period is seven days.

In accordance with another aspect of the present invention, a method for reducing a production of a slime in a papermaking process includes steps of adding a dispersing agent into the slime to obtain a first mixture, mixing and culturing the first mixture, adding an antagonistic into the first mixture to obtain a second mixture, and mixing and culturing the second mixture for reducing the production of the slime.

Preferably, the slime is produced during the papermaking process.

Preferably, the antagonistic is *Streptomyces bikiniensis.*

Preferably, the antagonistic is amounted to $10^7$/mL after being cultivated for 24 hrs.

Preferably, the dispersing agent is selected from a group consisting of a Lignosulfonate (B100), a Di-alkyl sulfosuccinate (S100), a Nonionic surfactants (H40), a Polyethylene glycol (P100), a Nonionic surfactants (Bu200).

In accordance with another aspect of the present invention, a mixture for reducing a production of a slime in a papermaking process include a dispersing agent, and an antagonistic, wherein the mixture and the slime are mixed and cultivated for reducing the production of the slime.

Preferably, the slime is produced during the papermaking process.

Preferably, the antagonistic is *Streptomyces bikiniensis.*

Preferably, the antagonistic is amounted to $10^7$/mL after being cultivated for 24 hrs.

Preferably, the dispersing agent is selected from a group consisting of a Lignosulfonate (B100), a Di-alkyl sulfosuccinate (S100), a Nonionic surfactants (H40), a Polyethylene glycol (P100), a Nonionic surfactants (Bu200).

The present invention may best be understood through the following descriptions with reference to the accompanying drawings, in which:

The present invention will now be described more specifically with reference to the following embodiments. It is to be noted that the following descriptions of preferred embodiments of this invention are presented herein for purpose of illustration and description only; it is not intended to be exhaustive or to be limited to the precise form disclosed.

Example 1: The effect of the antagonistic and the dispersing agent on the formation of the deposit

The experimental steps are described as follows:

Cultures which are isolated from the slime are cultivated in a NB cultivation liquid for 21-24 hours. Then, 1 mL culture liquids of the isolated cultures are centrifuged in 15000 rpm for 1 min and the supernatants thereof are removed. All the isolated cultures are re-suspended by adding 1 mL sterile water, and then each re-suspended culture is inoculated into a flask which already contains sterile white water 100 mL and 3 g LBKP pulp. Later, different dispersing agents are added into different flasks of each kind of culture to obtain different mixtures. The mixtures are cultivated in an incubate shaker at 40 °C, 70 rpm. After being mixture cultivated for 3 hours, 3 mL antagonistic C5 (*Streptomyces bikiniensis*) which has been amounted to $10^7$/mL after being cultivated for 24 hours is added thereinto to obtain another mixture. The mixture are cultivated in the incubate shaker again at 40 °C, 70 rpm. After 7 days, the production of the deposit is recorded.

The whole experiment is separated into six groups.

There are two contrast groups:

1: Only incubate the antagonistic C5 without incubating the culture isolated from the slime; and
2: Do not incubate any culture.

There are four experimental group:

3: Only incubate the culture isolated from the slime without incubating the antagonistic C5;
4: Incubate both the culture isolated from the slime and the antagonistic C5;
5: Incubate the culture isolated from the slime and the dispersing agent; and
6: Incubate the culture isolated from the slime, the dispersing agent, and the antagonistic C5.

The groups described above are double checked and all experiments are repeated at least once. Deposit % is calculated by the formula as follows:

$$\text{Deposit \%} = \frac{\text{Deposit dry weight of experimental group}}{\text{Deposit dry weight of the culture isolated from slime}} \times 100\%$$

Results:

The experimental results of "The effect of the antagonistic and the dispersing agent on the formation of the deposit" are shown in Table 1.

Table 1: The result of the effect of adding antagonistic and disperaing agent on the slime formation.

| Process method | Deposit (%) of the slime |
|---|---|
| Culture isolated from slime | 100.0 |
| Isolated culture + dispersing agent B 100 | 113.0 |
| Isolated culture + dispersing agent S100 | 53.0 |
| Isolated culture + dispersing agent H40 | 126.0 |
| Isolated culture + dispersing agent P100 | 112.0 |
| Isolated culture + dispersing agent Bu200 | 211.0 |
| Isolated culture + Antagonistic C5 | 53.0 |

EP 1 413 675 A1

(continued)

| Process method | Deposit (%) of the slime |
|---|---|
| Isolated culture + dispersing agent B100 + Antagonistic C5 | 74.6 |
| Isolated culture + dispersing agent S100 + Antagonistic C5 | 49.0 |
| Isolated culture + dispersing agent H40 + Antagonistic C5 | 46.0 |
| Isolated culture + dispersing agent P100 + Antagonistic C5 | 105.0 |
| Isolated culture + dispersing agent Bu200 + Antagonistic C5 | 66.4 |
| Antagonistic C5 | 24.7 |
| The contrast group without any additives | 12.0 |

Please refer to Table 1 which shows the experimental results of Example 1. Firstly, let's focus on the groups that are only added dispersing agent. Obviously, in the result of adding dispersing agent S100 (Di-alkyl sulfosuccinate), the deposit amount is reduced to 53%. Compared to the contrast group (100%) which is only added the culture isolated from the slime, the deposit amount is significantly different, namely, the dispersing agent (S100) has a good dispersing ability for the formation of the slime.

Moreover, in the groups which additionally add antagonistic C5 after adding the dispersing agent, the deposit amount is 74.6% when the dispersing agent is B 100 (Lignosulfonate), the deposit amount is 49% when the dispersing agent is S100, the deposit amount is 46% when the dispersing agent is H40 (Nonionic surfactants), the deposit amount is 105% when the dispersing agent is P100 (Polyethylene glycol), and the deposit amount is 66.4% when the dispersing agent is Bu200 (Nonionic surfactants). As shown above, these five groups show obvious reductions when compared to the contrast deposit 100%. According to the result, the addition of the antagonistic C5 is positive to inhibit/reduce the slime formation. Furthermore, no matter which dispersing agent is added with the antagonistic C5, after adding the antagonistic C5, the deposit amount can be further reduced actually. Thus, this experiment . improves that the antagonistic owns the ability to reduce the slime production.

Example 2: The track experiment of periodically adding the antagonistic

The experimental steps are described as follows:

Cultures which are isolated from the slime are cultivated in a NB cultivation liquid for 21-24 hours. Then, 1 mL culture liquids of the isolated cultures are centrifuged in 15000 rpm for 1 min and the supernatants thereof are removed. All the isolated cultures are re-suspended by adding 1 mL sterile water, and then each re-suspended culture is inoculated into a flask which already contains sterile white water 100 mL and 3 g LBKP pulp. Later, different dispersing agents are added into different flasks of each kind of culture to obtain different mixtures. The mixtures are cultivated in an incubate shaker at 40 °C, 70 rpm. After being mixture cultivated for 3 hours, 3 mL antagonistic C5 (*Streptomyces bikiniensis*) which has been amounted to $10^7$/mL after being cultivated for 24 hours is added thereinto to obtain another mixture. The mixture are cultivated in the incubate shaker again at 40 °C, 70 rpm. After 7 days, the production of the deposit is recorded and simultaneously the antagonistic C5 is re-added thereinto. Then, the final deposit amount after another 7 days (total 14 days) is also recorded.

The whole experiment is separated into four groups.

There are two contrast groups:

1: Only incubate the antagonistic C5 without incubating the culture isolated from the slime; and
2: Do not incubate any culture.
There are two experimental group:
3: Only incubate the culture isolated from the slime without incubating the antagonistic C5; and
4: Incubate both the culture isolated from the slime and the antagonistic C5.

The groups described above are double checked and all experiments are repeated at least once. Deposit % is calculated by the formula as follows:

$$\text{Deposit \%} = \frac{\text{Deposit dry weight of experimental group}}{\text{Deposit dry weight of the culture isolated from slime}} \times 100\%$$

Results:

The experimental results of "The track experiment of periodically adding the antagonistic" are shown in Table 2.

Table 2: The result of the track experiment of periodically adding the antagonistic

| Process method | Deposit % (Day 7) | Deposit % (Day 14) |
|---|---|---|
| Culture isolated from slime | 100.0 | 100.0 |
| Culture isolated from slime + Antagonistic C5 | 29.0 | 87.0 |
| Culture isolated from slime + Antagonistic C5 (Day 7) + Antagonistic C5 | | 28.0 |
| Antagonistic C5 | 31.0 | 35.0 |
| Contrast group without any additives | 27.0 | 24.0 |

Please refer to Table 2. In the group which contains the culture isolated from the slime, the deposit amount is 29% after adding the antagonistic C5 and cultivating for 7 days. Compared to the contrast deposit 100% which is only added the culture isolated from slime, the difference is strictly obvious. However, the difference will be gradually reduced corresponding to the increase of the cultivation time (the deposit amount is 87% when Day 14). But, if another antagonistic C5 is added at Day 7, the deposition at Day 14 (after another 7 days) will be still remained as 28% which is obviously different from the contrast deposit 100%. According to the result, the periodical addition (7 days) of the antagonistic C5 is positive to inhibit/reduce the adhesion of the deposit. Consequently, if it can utilize this method of adding the antagonistic C5 periodically to easily control the production of the slime, this will be a convenient and fast way for the industry. Furthermore, if take 7 days as a period, it will not be a highly concentrated time period, so that it will not consume too much labor and is really a practicable method.

In view of the aforesaid, through the proving of the experiments, the mixture of the dispersing agent and the local antagonistic and the method utilizing the same according to the present invention can actually and efficiently reduce the production of the slime during the papermaking process, solve the problems caused by the slime, such as paper holey, foxing, and paper break, and further improve the quality of the paper. Moreover, the method for reducing the slime production and the mixture making the same according to the present invention can substitute for the organic biocide and will not endanger the natural environment, so that it conforms to the standard of the environmental protection and the environmental consciousness. More importantly, the present invention can easily improve the slime problem in the original papermaking process without increasing the production costs. Consequently, this is really an invention with creativity and industrial value.

## Claims

1. A method for reducing a production of a slime in a papermaking process, comprising steps of:

    adding a dispersing agent into the slime to obtain a first mixture;
    mixing and culturing the mixture;
    adding an antagonistic into the first mixture to obtain a second mixture;
    mixing and culturing the second mixture for reducing the production of the slime; and
    re-adding the antagonistic into the mixed and cultured second mixture after a specific time period for further reducing the production of the slime.

2. The method according to claim 1, **characterized in that** the slime is produced during the papermaking process.

3. The method according to claim 1, **characterized in that** the antagonistic is *Streptomyces bikiniensis.*

4. The method according to claim 1, **characterized in that** the antagonistic is amounted to $10^7$/mL after being cultivated for 24 hrs.

5. The method according to claim 1, **characterized in that** the dispersing agent is selected from a group consisting of a Lignosulfonate (B100), a Di-alkyl sulfosuccinate (S 100), a Nonionic surfactants (H40), a Polyethylene glycol (P100), a Nonionic surfactants (Bu200).

6. The method according to claim 1, **characterized in that** the specific time period is seven days.

7. A method for reducing a production of a slime in a papermaking process, comprising steps of:

   adding a dispersing agent into the slime to obtain a first mixture;
   mixing and culturing the first mixture;
   adding an antagonistic into the first mixture to obtain a second mixture; and
   mixing and culturing the second mixture for reducing the production of the slime.

8. The method according to claim 7, **characterized in that** the slime is produced during the papermaking process.

9. The method according to claim 7, **characterized in that** the antagonistic is *Streptomyces bikiniensis.*

10. The method according to claim 7, **characterized in that** the antagonistic is amounted to $10^7$/mL after being cultivated for 24 hrs.

11. The method according to claim 7, **characterized in that** the dispersing agent is selected from a group consisting of a Lignosulfonate (B 100), a Di-alkyl sulfosuccinate (S100), a Nonionic surfactants (H40), a Polyethylene glycol (P100), a Nonionic surfactants (Bu200).

12. A mixture for reducing a production of a slime in a papermaking process, comprising:

    a dispersing agent; and
    an antagonistic,

    **characterized in that** the mixture and the slime are mixed and cultivated for reducing the production of the slime.

13. The mixture according to claim 12, **characterized in that** the slime is produced during the papermaking process.

14. The mixture according to claim 12, **characterized in that** the antagonistic is *Streptomyces bikiniensis.*

15. The mixture according to claim 12, **characterized in that** the antagonistic is amounted to $10^7$/mL after being cultivated for 24 hrs.

16. The mixture according to claim 12, **characterized in that** the dispersing agent is selected from a group consisting of a Lignosulfonate (B100), a Di-alkyl sulfosuccinate (S100), a Nonionic surfactants (H40), a Polyethylene glycol (P100), a Nonionic surfactants (Bu200).

**European Patent Office**

# EUROPEAN SEARCH REPORT

Application Number

EP 03 00 5362

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | US 6 281 002 B1 (MOELLER-BREMER CHRISTINE) 28 August 2001 (2001-08-28) | 7,12 | D21H21/04 |
| A | * claims 1-10 * | 1-6, 8-11, 13-16 | |
| | --- | | |
| X | EP 0 338 439 A (KATAYAMA CHEMICAL WORKS CO) 25 October 1989 (1989-10-25) * claims 1-13; example 1 * | 1,7,12 | |
| | --- | | |
| X | WO 96 31610 A (SPEYBROECK MICHEL M P VAN ;BRUGGEMAN GEERT (BE); POELE JOZEF VAN ()) 10 October 1996 (1996-10-10) * claims 1-20; examples 1-14 * | 7,12 | |
| | --- | | |
| X | WO 99 44424 A (BUCKMAN LABOR INC) 10 September 1999 (1999-09-10) | 12 | |
| Y | * claims 1-11 * | 7 | |
| | --- | | |
| X | US 5 874 453 A (KING VANJA M ET AL) 23 February 1999 (1999-02-23) | 12 | TECHNICAL FIELDS SEARCHED (Int.Cl.7) |
| Y | * claims 1-32 * | 7 | D21H |
| | --- | | C02F |
| A | EP 1 114 892 A (SK CORP) 11 July 2001 (2001-07-11) * the whole document * | 1-16 | |
| | --- | | |
| A | US 5 256 182 A (FRIEDMAN JR LESTER A ET AL) 26 October 1993 (1993-10-26) * the whole document * | 1-16 | |
| | ----- | | |
| | ＊ | | |

The present search report has been drawn up for all claims

| Place of search | Date of completion of the search | Examiner |
|---|---|---|
| MUNICH | 29 August 2003 | Karlsson, L |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

## ANNEX TO THE EUROPEAN SEARCH REPORT
## ON EUROPEAN PATENT APPLICATION NO.

EP 03 00 5362

This annex lists the patent family members relating to the patent documents cited in the above-mentioned European search report.
The members are as contained in the European Patent Office EDP file on
The European Patent Office is in no way liable for these particulars which are merely given for the purpose of information.

29-08-2003

| Patent document cited in search report | | Publication date | Patent family member(s) | | Publication date |
|---|---|---|---|---|---|
| US 6281002 | B1 | 28-08-2001 | DE | 4421504 A1 | 21-12-1995 |
| | | | AT | 209441 T | 15-12-2001 |
| | | | CA | 2151996 A1 | 21-12-1995 |
| | | | DE | 59509876 D1 | 10-01-2002 |
| | | | EP | 0688500 A1 | 27-12-1995 |
| | | | ES | 2166792 T3 | 01-05-2002 |
| | | | FI | 953018 A | 21-12-1995 |
| EP 0338439 | A | 25-10-1989 | CA | 1307202 C | 08-09-1992 |
| | | | CA | 1307203 C | 08-09-1992 |
| | | | DE | 68901206 D1 | 21-05-1992 |
| | | | DE | 68903664 D1 | 14-01-1993 |
| | | | DE | 68903664 T2 | 08-04-1993 |
| | | | EP | 0338439 A1 | 25-10-1989 |
| | | | EP | 0338440 A1 | 25-10-1989 |
| | | | FI | 891812 A ,B, | 19-10-1989 |
| | | | FI | 891813 A ,B, | 19-10-1989 |
| | | | JP | 2042007 A | 13-02-1990 |
| | | | JP | 2716044 B2 | 18-02-1998 |
| | | | US | 4963586 A | 16-10-1990 |
| | | | US | 5026723 A | 25-06-1991 |
| WO 9631610 | A | 10-10-1996 | AT | 214099 T | 15-03-2002 |
| | | | AU | 705804 B2 | 03-06-1999 |
| | | | AU | 5500296 A | 23-10-1996 |
| | | | CA | 2215996 A1 | 10-10-1996 |
| | | | DE | 69619665 D1 | 11-04-2002 |
| | | | DE | 69619665 T2 | 31-10-2002 |
| | | | WO | 9631610 A2 | 10-10-1996 |
| | | | EP | 0820516 A2 | 28-01-1998 |
| | | | ES | 2174068 T3 | 01-11-2002 |
| | | | JP | 11503317 T | 26-03-1999 |
| | | | NO | 974545 A | 07-10-1997 |
| | | | NZ | 306445 A | 25-02-1999 |
| | | | ZA | 9602723 A | 04-12-1996 |
| WO 9944424 | A | 10-09-1999 | AU | 746012 B2 | 11-04-2002 |
| | | | AU | 2899799 A | 20-09-1999 |
| | | | BR | 9908791 A | 31-10-2000 |
| | | | CA | 2322695 A1 | 10-09-1999 |
| | | | EP | 1059844 A1 | 20-12-2000 |
| | | | JP | 2002506006 T | 26-02-2002 |
| | | | NZ | 506589 A | 28-02-2003 |
| | | | WO | 9944424 A1 | 10-09-1999 |
| | | | ZA | 9901787 A | 27-10-1999 |

EPO FORM P0459

For more details about this annex : see Official Journal of the European Patent Office, No. 12/82

## ANNEX TO THE EUROPEAN SEARCH REPORT
## ON EUROPEAN PATENT APPLICATION NO.

EP 03 00 5362

This annex lists the patent family members relating to the patent documents cited in the above-mentioned European search report.
The members are as contained in the European Patent Office EDP file on
The European Patent Office is in no way liable for these particulars which are merely given for the purpose of information.

29-08-2003

| Patent document cited in search report | | Publication date | Patent family member(s) | | Publication date |
|---|---|---|---|---|---|
| US 5874453 | A | 23-02-1999 | AU | 725645 B2 | 19-10-2000 |
| | | | AU | 8276598 A | 08-02-1999 |
| | | | BR | 9810567 A | 19-09-2000 |
| | | | CN | 1262601 T | 09-08-2000 |
| | | | EP | 0994651 A1 | 26-04-2000 |
| | | | JP | 2001509474 T | 24-07-2001 |
| | | | NZ | 502239 A | 20-12-2002 |
| | | | WO | 9902037 A1 | 21-01-1999 |
| EP 1114892 | A | 11-07-2001 | KR | 2001063993 A | 09-07-2001 |
| | | | EP | 1114892 A1 | 11-07-2001 |
| | | | JP | 2001181997 A | 03-07-2001 |
| | | | US | 6380174 B1 | 30-04-2002 |
| US 5256182 | A | 26-10-1993 | US | 4975109 A | 04-12-1990 |
| | | | CA | 1329351 C | 10-05-1994 |
| | | | EP | 0555210 A1 | 18-08-1993 |
| | | | NO | 931506 A | 21-06-1993 |
| | | | WO | 9207465 A1 | 14-05-1992 |